# EUROPEAN PATENT APPLICATION

(11) **EP 4 681 653 A1**
(43) Date of publication of application: **21.01.2026**
(21) Application number: 24770335.8
(22) Date of filing: 09.02.2024
(51) Int. Cl.: A61B 8/08

(54) **ULTRASONIC DIAGNOSTIC DEVICE AND METHOD FOR CONTROLLING ULTRASONIC DIAGNOSTIC DEVICE**

(30) Priority: 15.03.2023 JP 2023041083
(71) Applicant: FUJIFILM Corporation, Tokyo 106-8620 (JP)
(72) Inventor: SATO Ryosuke, Tokyo 106-8620 (JP)
(74) Representative: Klunker IP Patentanwälte PartG mbB
(86) International application number: PCT/JP2024/004572
(87) International publication number: WO 2024/190217

(57) **Abstract**

An ultrasound diagnostic apparatus includes: an image acquisition unit (33) that continuously acquires ultrasound images of a plurality of frames in which a mammary gland region of a subject is imaged; a lesion detection unit (26) that detects a suspected lesion region in the mammary gland region for each of the ultrasound images of the plurality of frames; a target frame generation unit (27) that generates an evaluation target frame group with ultrasound images of frames other than a frame in which the suspected lesion region is detected among the ultrasound images of the plurality of frames; and an evaluation unit (29) that performs a glandular tissue component evaluation on the ultrasound image of each frame in the evaluation target frame group.

## Description

### BACKGROUND OF THE INVENTION

### 1. Field of the Invention

The present invention relates to an ultrasound diagnostic apparatus used for an examination of a breast of a subject and a method of controlling the ultrasound diagnostic apparatus.

### 2. Description of the Related Art

In the related art, an ultrasound diagnostic apparatus that captures an ultrasound image of a subject has been put into practical use in the medical field. In general, the ultrasound diagnostic apparatus comprises an ultrasound probe provided with a transducer array built therein and an apparatus body connected to the ultrasound probe, in which an ultrasound beam is transmitted from the ultrasound probe toward a subject, an ultrasound echo from the subject is received by the ultrasound probe, and a reception signal is electrically processed to generate the ultrasound image.

A composition of fat and a mammary gland tissue of a breast varies depending on a person, but an anatomical structure of the breast is common, and in the mammary gland tissue, a main mammary duct branches into a large number of extralobular ducts, which are connected to a large number of lobules. Stroma is present around the lobules, and mammary gland tissue is composed of the lobules together with the stroma.

It is known that two types of stroma exist around the lobules, that is, perilobular stroma and edematous stroma. The perilobular stroma exists along a structure from the lobule to the mammary duct and includes many collagen fibers. Meanwhile, the edematous stroma fills the spaces between the perilobular stroma, is rich in extracellular matrix, with a mixture of collagen fibers and fat, and contains fewer collagen fibers as compared to the perilobular stroma.

In recent years, the concept of individualized risk management for patients has become widespread, but it is known that a ratio of the mammary gland region within the breast, especially a high-density mammary gland, is a risk factor for cancer. The ratio of the mammary gland region in the breast can be measured by using a mammography apparatus.

In Su Hyun Lee et al. "Glandular Tissue Component and Breast Cancer Risk in Mammographically Dense Breasts at Screening Breast US", Radiology, Volume 301, October 1, 2021, it is reported that a cancer is likely to occur in a case in which a ratio of a glandular tissue component (GTC) region including mammary ducts, lobules, and perilobular stroma in the mammary gland region is high even though the mammary gland region is almost the same. That is, in addition to the ratio of the mammary gland region in the breast, a ratio of the GTC region in the mammary gland region may be a risk factor. This means a higher risk in a patient with less advanced atrophy of the lobule.

However, in the mammography apparatus, the perilobular stroma and the edematous stroma cannot be distinguished from each other, and the entire mammary gland tissue is observed as whitish, and as a result, the ratio of the GTC region in the mammary gland region cannot be measured.

WO2018/180386A discloses an ultrasound diagnostic apparatus that extracts a suspected lesion region in a mammary gland region, which is a region suspected to have a lesion.

### SUMMARY OF THE INVENTION

However, the ultrasound diagnostic apparatus disclosed in WO2018/180386A is intended to detect the suspected lesion region in the mammary gland region, and is not interested in evaluating the GTC region. Therefore, there is an issue in that the risk of cancer in the mammary gland region cannot be considered in detail.

In addition, since both the GTC region and the suspected lesion region are depicted as a low-echo region, that is, a low-brightness region in the ultrasound image, in a case in which the GTC evaluation is performed based on the ultrasound image of the frame including the suspected lesion region, an accurate evaluation result cannot be obtained, and the user such as a doctor may not be able to accurately consider the risk of cancer in the mammary gland region.

The present invention has been made in order to solve this issue in the related art, and an object of the present invention is to provide an ultrasound diagnostic apparatus that enables a user to consider a risk of cancer in a mammary gland region of a subject with high accuracy even in a case in which a suspected lesion region is present.

It is possible to achieve the above-described object with the following configurations.
[1] An ultrasound diagnostic apparatus comprising: an image acquisition unit that continuously acquires ultrasound images of a plurality of frames in which a mammary gland region of a subject is imaged; a lesion detection unit that detects a suspected lesion region in the mammary gland region for each of the ultrasound images of the plurality of frames; a target frame generation unit that generates an evaluation target frame group with ultrasound images of frames other than a frame in which the suspected lesion region is detected by the lesion detection unit among the ultrasound images of the plurality of frames; and an evaluation unit that performs a glandular tissue component evaluation on the ultrasound image of each frame in the evaluation target frame group.
[2] The ultrasound diagnostic apparatus according to [1], in which the target frame generation unit generates the evaluation target frame group with ultrasound images of frames on and before a frame that is a predetermined number of frames before the frame in which the suspected lesion region is detected by the lesion detection unit and ultrasound images of frames on and after a frame that is a predetermined number of frames after the frame in which the suspected lesion region is detected by the lesion detection unit, among the ultrasound images of the plurality of frames.
[3] The ultrasound diagnostic apparatus according to [1] or [2], further comprising: a size calculation unit that calculates a size of the suspected lesion region detected by the lesion detection unit, in which the target frame generation unit includes, in the evaluation target frame group, an ultrasound image of a frame in which the size of the suspected lesion region calculated by the size calculation unit is less than a predetermined size threshold value.
[4] The ultrasound diagnostic apparatus according to any one of [1] to [3], further comprising: a malignancy degree calculation unit that calculates a malignancy degree of the suspected lesion region detected by the lesion detection unit, in which the target frame generation unit includes, in the evaluation target frame group, an ultrasound image of a frame in which the malignancy degree of the suspected lesion region calculated by the malignancy degree calculation unit is less than a predetermined malignancy degree threshold value.
[5] The ultrasound diagnostic apparatus according to any one of [1] to [4], further comprising: a monitor; and a display control unit that displays the ultrasound images of the plurality of frames on the monitor.
[6] The ultrasound diagnostic apparatus according to [5], in which the display control unit displays the ultrasound image of each frame in the evaluation target frame group generated by the target frame generation unit, on the monitor.
[7] The ultrasound diagnostic apparatus according to [6], in which the evaluation unit performs the glandular tissue component evaluation on an ultrasound image of a frame designated by a user in the evaluation target frame group.
[8] The ultrasound diagnostic apparatus according to any one of [5] to [7], in which the display control unit displays an ultrasound image of the frame in which the suspected lesion region is detected by the lesion detection unit among the ultrasound images of the plurality of frames, on the monitor.
[9] The ultrasound diagnostic apparatus according to [8], in which the display control unit highlights the suspected lesion region on the monitor.
[10] The ultrasound diagnostic apparatus according to any one of [5] to [7], in which the display control unit displays an ultrasound image of each frame in which processing of detecting the suspected lesion region is performed by the lesion detection unit, on the monitor.
[11] The ultrasound diagnostic apparatus according to [10], in which the display control unit displays a dialog for confirming with a user whether or not to correct a detection result of the suspected lesion region obtained by the lesion detection unit, on the monitor.
[12] The ultrasound diagnostic apparatus according to any one of [5] to [11], in which the evaluation unit classifies the mammary gland region of the ultrasound image of each frame in the evaluation target frame group into a low-echo region and a high-echo region based on a predetermined brightness threshold value, and displays a glandular tissue component ratio represented by a ratio between the number of pixels occupied by the low-echo region and the number of pixels occupied by the high-echo region, on the monitor as a result of the glandular tissue component evaluation.
[13] The ultrasound diagnostic apparatus according to [12], in which the evaluation unit displays any one of an average value, a median value, or a maximum value of the glandular tissue component ratios calculated in each frame in the evaluation target frame group, or any one of an average value, a median value, or a maximum value of the glandular tissue component ratios obtained by excluding outliers from the glandular tissue component ratios calculated in each frame in the evaluation target frame group, on the monitor as the result of the glandular tissue component evaluation.
[14] The ultrasound diagnostic apparatus according to any one of [5] to [11], in which the evaluation unit determines a category of a glandular tissue component in the mammary gland region based on the ultrasound images in the evaluation target frame group, and displays the category on the monitor as a result of the glandular tissue component evaluation.
[15] The ultrasound diagnostic apparatus according to any one of [1] to [14], in which the lesion detection unit detects the suspected lesion region using a trained model that has been trained through machine learning based on a plurality of training data each of which includes the ultrasound image in which the mammary gland region including the suspected lesion region is imaged.
[16] The ultrasound diagnostic apparatus according to any one of [1] to [14], in which the lesion detection unit detects the suspected lesion region by image-analyzing the ultrasound image.
[17] A method of controlling an ultrasound diagnostic apparatus, the method comprising: continuously acquiring ultrasound images of a plurality of frames in which a mammary gland region of a subject is imaged; detecting a suspected lesion region in the mammary gland region for each of the ultrasound images of the plurality of frames; generating an evaluation target frame group with ultrasound images of frames other than a frame in which the suspected lesion region is detected among the ultrasound images of the plurality of frames; and performing a glandular tissue component evaluation on the ultrasound image of each frame in the evaluation target frame group.

According to the aspects of the present invention, the ultrasound diagnostic apparatus comprises the image acquisition unit that continuously acquires the ultrasound images of the plurality of frames in which the mammary gland region of the subject is imaged; the lesion detection unit that detects the suspected lesion region in the mammary gland region for each of the ultrasound images of the plurality of frames; the target frame generation unit that generates the evaluation target frame group with the ultrasound images of the frames other than the frame in which the suspected lesion region is detected by the lesion detection unit among the ultrasound images of the plurality of frames; and the evaluation unit that performs the glandular tissue component evaluation on the ultrasound image of each frame in the evaluation target frame group, so that the user can accurately assess the risk of cancer in the mammary gland region of the subject even in a case in which the suspected lesion region is present.

### BRIEF DESCRIPTION OF THE DRAWINGS

Fig. 1 is a block diagram showing a configuration of an ultrasound diagnostic apparatus according to Embodiment 1 of the present invention.
Fig. 2 is a block diagram showing an internal configuration of a transmission-and-reception circuit according to Embodiment 1.
Fig. 3 is a block diagram showing an internal configuration of an image generation unit according to Embodiment 1.
Fig. 4 is a diagram showing an ultrasound image obtained by imaging a mammary gland region of a subject.
Fig. 5 is a diagram showing an ultrasound image including a mammary gland region in which a GTC region is imaged.
Fig. 6 is a diagram schematically showing an example of an evaluation target frame group.
Fig. 7 is a diagram showing a display example of an evaluation result of a GTC evaluation.
Fig. 8 is a flowchart showing an operation according to Embodiment 1.
Fig. 9 is a diagram showing a display example of the evaluation target frame group selected by a user.
Fig. 10 is a diagram showing a display example of an exclusion frame confirmation button.
Fig. 11 is a diagram showing a display example of the ultrasound image in which a suspected lesion region is detected.
Fig. 12 is a block diagram showing a configuration of an ultrasound diagnostic apparatus according to Embodiment 2 of the present invention.
Fig. 13 is a flowchart showing an operation according to Embodiment 2.
Fig. 14 is a block diagram showing a configuration of an ultrasound diagnostic apparatus according to Embodiment 3 of the present invention.
Fig. 15 is a flowchart showing an operation according to Embodiment 3.

### DESCRIPTION OF THE PREFERRED EMBODIMENTS

Hereinafter, embodiments of the present invention will be described with reference to the accompanying drawings.

The following configuration requirements are described based on a representative embodiment of the present invention, but the present invention is not limited to such an embodiment.

In addition, the present specification, a numerical range represented by "to" means a range including numerical values described before and after "to", both ends inclusive, as a lower limit value and an upper limit value.

In the present specification, "same" and "identical" include an error range that is generally allowed in the technical field.

### [Embodiment 1]

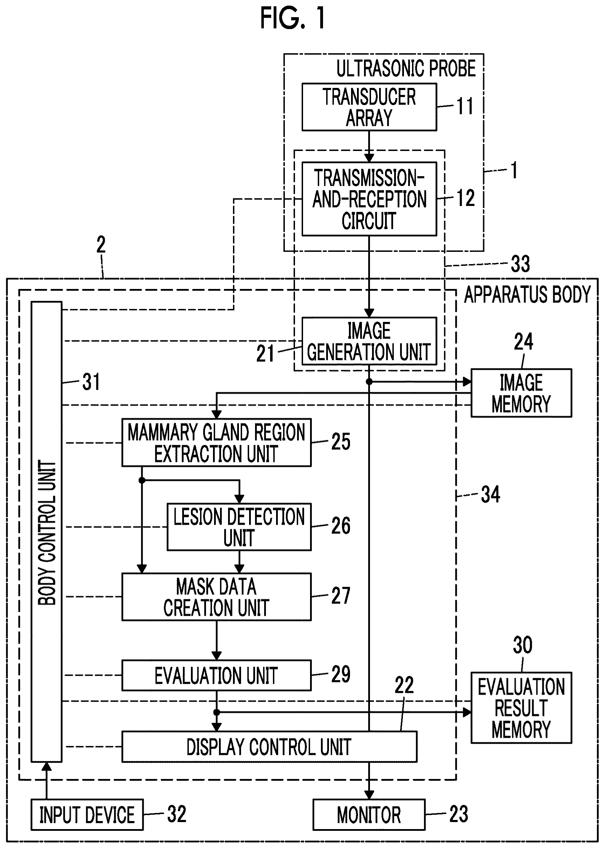

Fig. 1 shows a configuration of an ultrasound diagnostic apparatus according to Embodiment 1 of the present invention. The ultrasound diagnostic apparatus comprises an ultrasound probe 1 and an apparatus body 2. The ultrasound probe 1 and the apparatus body 2 are wired-connected to each other through a cable (not shown).

The ultrasound probe 1 includes a transducer array 11 and a transmission-and-reception circuit 12 connected to the transducer array 11.

The apparatus body 2 includes an image generation unit 21 connected to the transmission-and-reception circuit 12 of the ultrasound probe 1, a display control unit 22 and a monitor 23 are connected sequentially to the image generation unit 21, and an image memory 24 is connected to the image generation unit 21. A mammary gland region extraction unit 25 is connected to the image memory 24. A lesion detection unit 26 is sequentially connected to the mammary gland region extraction unit 25. Further, a target frame generation unit 27 is connected to the mammary gland region extraction unit 25 and the lesion detection unit 26. An evaluation unit 29 is connected to the target frame generation unit 27. The display control unit 22 and an evaluation result memory 30 are connected to the evaluation unit 29.

Further, a body control unit 31 is connected to the image generation unit 21, the display control unit 22, the image memory 24, the mammary gland region extraction unit 25, the target frame generation unit 27, the evaluation unit 29, and the evaluation result memory 30. An input device 32 is connected to the body control unit 31. The transmission-and-reception circuit 12 and the image generation unit 21 constitute an image acquisition unit 33. The image generation unit 21, the display control unit 22, the mammary gland region extraction unit 25, the lesion detection unit 26, the target frame generation unit 27, the evaluation unit 29, and the body control unit 31 constitute a processor 34 for the apparatus body 2.

The transducer array 11 of the ultrasound probe 1 includes a plurality of ultrasound transducers arranged in a one-dimensional or two-dimensional manner. Each of these transducers transmits an ultrasound wave in response to a drive signal supplied from the transmission-and-reception circuit 12, receives a reflected wave from a subject, and outputs an analog reception signal. Each transducer is formed by, for example, forming electrodes on both ends of a piezoelectric body consisting of a piezoelectric single crystal represented by lead zirconate titanate (PZT), a polymeric piezoelectric element represented by poly vinylidene di fluoride (PVDF), or a piezoelectric single crystal represented by a lead magnesium niobate-lead titanate (PMN-PT) solid solution.

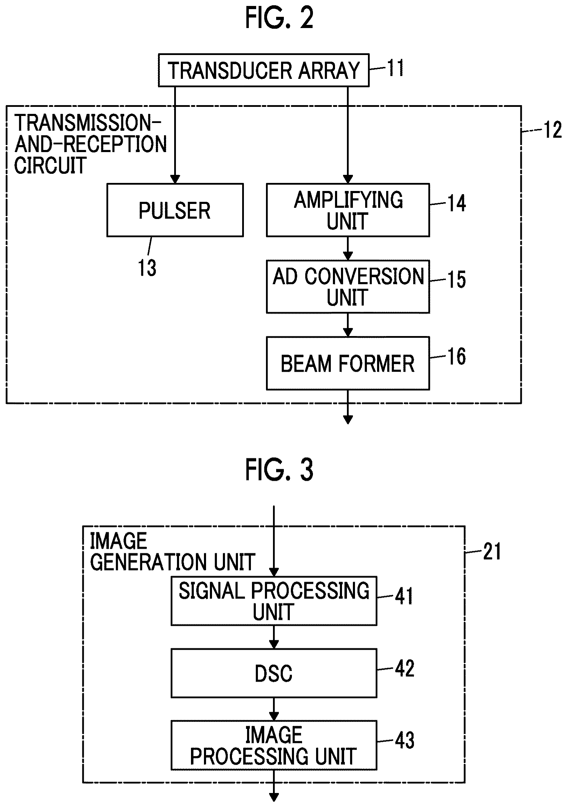

The transmission-and-reception circuit 12 transmits the ultrasound wave from the transducer array 11 and generates a sound ray signal based on the reception signal acquired by the transducer array 11, under the control of the body control unit 31. The transmission-and-reception circuit 12 includes, as shown in Fig. 2, a pulser 13 connected to the transducer array 11, and an amplifying unit 14, an analog-digital (AD) conversion unit 15, and a beam former 16 which are sequentially connected in series to the transducer array 11.

The pulser 13 includes, for example, a plurality of pulse generators, adjusts a delay amount of each drive signal based on a transmission delay pattern selected in accordance with a control signal from the body control unit 31 such that ultrasound waves to be transmitted from the plurality of transducers of the transducer array 11 form a ultrasound beam, and supplies the drive signal of which the delay amount has been adjusted, to the plurality of transducers. As described above, in a case in which a pulsed or continuous wave voltage is applied to the electrodes of the transducers of the transducer array 11, the piezoelectric body expands and contracts to generate a pulsed or continuous wave ultrasound wave from each transducer, and the ultrasound beam is formed from the combined wave of these ultrasound waves.

The transmitted ultrasound beam is, for example, reflected by a target such as a part of the subject, and an ultrasound echo propagates toward the transducer array 11 of the ultrasound probe 1. The ultrasound echo propagating toward the transducer array 11 in this manner is received by each of the transducers constituting the transducer array 11. In such a case, each transducer constituting the transducer array 11 expands and contracts by receiving the propagating ultrasound echo to generate the reception signal that is an electric signal, and outputs the reception signal to the amplifying unit 14.

The amplifying unit 14 amplifies the signal input from each of the transducers constituting the transducer array 11 and transmits the amplified signal to the AD conversion unit 15. The AD conversion unit 15 converts the signal transmitted from the amplifying unit 14 into digital reception data, and transmits the reception data to the beam former 16. The beam former 16 performs so-called reception focus processing by giving and adding delay with respect to each reception data converted by the AD conversion unit 15, in accordance with a sound velocity or a sound velocity distribution set based on a reception delay pattern selected according to a control signal from the body control unit 31. By the reception focus processing, a sound ray signal is acquired in which each piece of the reception data converted by the AD conversion unit 15 is phased and added and the focus of the ultrasound echo is narrowed.

The image generation unit 21 of the apparatus body 2 has, as shown in Fig. 3, a configuration in which a signal processing unit 41, a digital scan converter (DSC) 42, and an image processing unit 43 are sequentially connected in series.

The signal processing unit 41 performs, on the sound ray signal transmitted from the transmission-and-reception circuit 12 of the ultrasound probe 1, correction of attenuation caused by a distance in accordance with a depth of a reflection position of the ultrasound wave and then performs envelope detection processing, and thereby generates an ultrasound image signal (B-mode image signal), which is tomographic image information related to tissues in the subject.

The DSC 42 converts (raster-converts) the ultrasound image signal generated by the signal processing unit 41 into an image signal in accordance with a normal television signal scanning method.

The image processing unit 43 performs various types of necessary image processing, such as gradation processing, on the ultrasound image signal input from the DSC 42, and then outputs the signal representing the ultrasound image to the display control unit 22 and the image memory 24. The signal representing the ultrasound image generated by the image generation unit 21 in this way will be simply referred to as the ultrasound image. The image generation unit 21 can also output the ultrasound image signal before being processed by the DSC 42 or the ultrasound image signal immediately after being processed by the DSC 42 to the image memory 24. In this case, the image generation unit 21 can generate the ultrasound image by reading out these signals from the image memory 24 and performing processing using the DSC 42 or the image processing unit 43.

The image memory 24 is a memory that stores the ultrasound image generated by the image generation unit 21 under the control of the body control unit 31. For example, the image memory 24 can store a plurality of frames of ultrasound images generated by the image generation unit 21 in correspondence with diagnosis on a mammary gland region of a breast of the subject.

As the image memory 24, for example, a recording medium such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), or a universal serial bus memory (USB memory), can be used.

The mammary gland region extraction unit 25 detects a breast region of the subject from each of the ultrasound images of the plurality of frames read out from the image memory 24, and extracts the mammary gland region from the detected breast region.

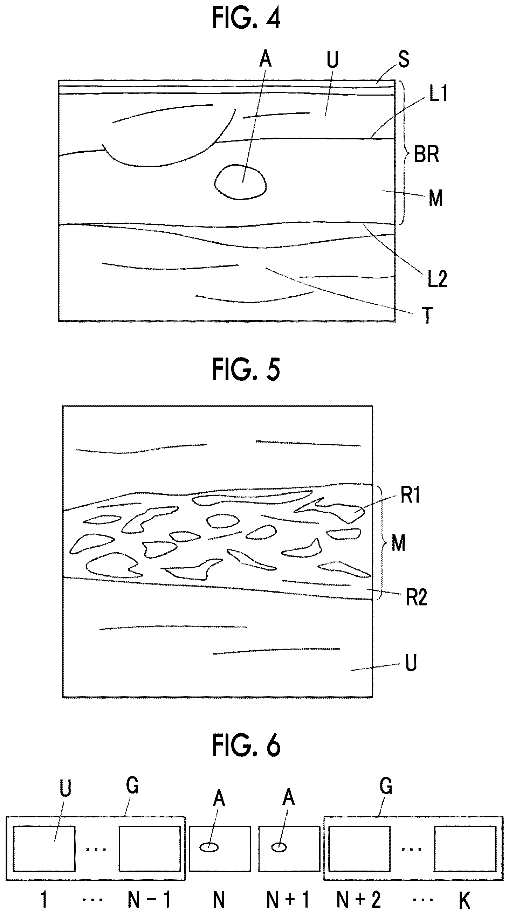

Fig. 4 shows an example of an ultrasound image U in which the breast of the subject is imaged. The ultrasound image U is a tomographic image captured by bringing a distal end of the ultrasound probe 1 into contact with the breast of the subject, in which a skin S of the subject is shown in an upper end of the ultrasound image U representing a shallowest portion, and a pectoralis major T is shown in a lower portion of the ultrasound image U representing a deeper portion. The mammary gland region extraction unit 25 can recognize a skin S and a pectoralis major T from the ultrasound image U and detect a deep region between the skin S and the pectoralis major T as a breast region BR.

As shown in Fig. 4, the mammary gland region extraction unit 25 can recognize a front boundary line L1 located on a shallower side and a rear boundary line L2 located on a deeper side in the detected breast region BR, and can extract a deep region between the front boundary line L1 and the rear boundary line L2 as a mammary gland region M.

In order to detect the breast region BR and to extract the mammary gland region M described above, the mammary gland region extraction unit 25 can perform image recognition using at least one of template matching, an image analysis technique using a feature value, such as adaptive boosting (Adaboost), support vector machine (SVM), or scale-invariant feature transform (SIFT), or a determination model that has been trained by using a machine learning technique such as deep learning.

The determination model is a trained model that has learned the breast region BR and the mammary gland region M (segmentation) of the breast region BR in a training ultrasound image obtained by imaging the breast.

The lesion detection unit 26 detects a suspected lesion region A based on the ultrasound image U in which the mammary gland region M of the subject is imaged. Here, the suspected lesion region A means a region in which a lesion including a so-called tumor is suspected in the mammary gland region M. The lesion detection unit 26 can detect the suspected lesion region A by using, for example, at least one of template matching, an image analysis technique using a feature value, such as Adaboost, SVM, or SIFT, or a determination model that has been trained by using a machine learning technique such as deep learning. The determination model used here is a trained model that has learned a plurality of lesion parts in the ultrasound image U in which the mammary gland region M is imaged.

In addition, the lesion detection unit 26 can assign a flag for excluding the ultrasound image U of the frame in which the suspected lesion region A is detected, from candidates of an evaluation target frame group, which will be described later.

As shown in Fig. 6, the target frame generation unit 27 generates an evaluation target frame group G with the ultrasound images U of frames other than the frame in which the suspected lesion region A is detected by the lesion detection unit 26 among the ultrasound images U of the plurality of frames generated by the image generation unit 21. For example, the target frame generation unit 27 can exclude the ultrasound image U of the frame to which the flag is assigned by the lesion detection unit 26 among the ultrasound images U of the plurality of frames, from the candidates of the evaluation target frame group G, to generate the evaluation target frame group G with the ultrasound images U of the frames to which the flag is not assigned. In the example of Fig. 6, the suspected lesion region A is detected in the ultrasound images U of an N-th frame and an (N+1)-th frame among the ultrasound images U of K frames, and the evaluation target frame group G is generated with the ultrasound images U of a first frame to an (N-1)-th frame and an (N+2)-th frame to a K-th frame in which the suspected lesion region A is not detected. It should be noted that N and K are natural numbers satisfying N < K.

The evaluation unit 29 performs a glandular tissue component (GTC) evaluation on the ultrasound image U of each frame in the evaluation target frame group G generated by the target frame generation unit 27.

In a case of performing the GTC evaluation, the evaluation unit 29 first extracts a GTC region R1 from the mammary gland region M extracted by the mammary gland region extraction unit 25 as shown in Fig. 5. The GTC region R1 consists of mammary ducts, lobules, and perilobular stroma in the mammary gland region M, and edematous stroma R2 fills spaces between the perilobular stroma. Since the edematous stroma R2 is rich in extracellular matrix and contains coexisting fat, in a case of observing the mammary gland region M using the ultrasound image U, the edematous stroma R2 has a relatively high echo level (high-echo) and appears bright. On the other hand, the mammary ducts, the lobules, and the perilobular stroma constituting the GTC region R1 have relatively low echo levels (low-echo), and have lower brightness than the edematous stroma R2.

Therefore, the evaluation unit 29 can classify the mammary gland region M of the ultrasound image U into a low-echo region and a high-echo region by, for example, binarizing the mammary gland region M using a brightness threshold value Thb, and extract the GTC region R1 by distinguishing the GTC region R1 and the edematous stroma R2 from each other in the mammary gland region M.

In addition, a predetermined constant value can be used as the brightness threshold value Thb.

In addition, the evaluation unit 29 may perform edge detection on the GTC region R1 in the ultrasound image U by image analysis, and may automatically calculate the brightness threshold value Thb based on a change in brightness value in the detected edge portion, that is, a change in brightness value of a plurality of pixels from the inside to the outside of the GTC region R1. In this way, it is possible to automatically set the brightness threshold value Thb suitable for the ultrasound image U as the image analysis target, and to acquire the binarized image suitable for the ultrasound image U.

Furthermore, the ultrasound diagnostic apparatus can be configured such that a histogram of the brightness of the mammary gland region M detected from the ultrasound image U is created, and the user sets the brightness threshold value Thb by inputting the brightness threshold value Thb from the input device 32 based on the histogram, a binarized image created using the initial value of the brightness threshold value Thb, and the ultrasound image U generated by the image generation unit 21.

In addition, the evaluation unit 29 can also extract the GTC region R1 using a determination model that has been trained by using a machine learning technique such as deep learning. In this case, for example, a trained model that has learned the GTC region R1 (segmentation) in the mammary gland region M in the training ultrasound image in which the breast is imaged is used as the determination model.

The evaluation unit 29 calculates a ratio of the GTC region R1 in the mammary gland region M, to perform the GTC evaluation based on the calculated ratio of the GTC region R1. The evaluation unit 29 can calculate the ratio of the GTC region R1, for example, by a ratio of the sum of the number of pixels occupied by all the low-echo regions in the mammary gland region M to the number of pixels occupied by the high-echo region in the mammary gland region M in the ultrasound image U.

The evaluation unit 29 can use, for example, any one of an average value, a median value, or a maximum value of the ratios of a predetermined number of GTC regions R1 calculated from the ultrasound images U of a predetermined number of frames as a final evaluation result of the GTC evaluation. In addition, the evaluation unit 29 can use any one of an average value, a median value, or a maximum value of the remaining GTC regions R1 as the final evaluation result of the GTC evaluation after excluding outliers from the ratio of the predetermined number of GTC regions R1.

The outlier means a value in which a difference between a plurality of values is larger than a predetermined difference threshold value. Further, the number of frames of the ultrasound image U used for the GTC evaluation can be determined in advance, for example, by an input operation performed by the user through the input device 32.

For example, the evaluation unit 29 can determine a category of the GTC region R1 based on the ratio of the GTC region R1 in the mammary gland region M and use the category as the evaluation result of the GTC evaluation.

It is generally known that the lobule atrophies with age, but there are research results that a risk of breast cancer is high in patients in whom the lobule does not atrophy, as disclosed in, for example, "Su Hyun Lee et al. "Glandular Tissue Component and Breast Cancer Risk in Mammographically Dense Breasts at Screening Breast US", Radiology, Volume 301, October 1,2021". The category of the GTC region R1 represents a degree of progression of the atrophy of the lobule, and can be used as a material for determining the risk of breast cancer.

The evaluation unit 29 can also determine the category of the GTC region R1 using, for example, a trained model that has been trained through machine learning based on a plurality of training data each of which includes the ultrasound image U in which the mammary gland region M is imaged and the category of the GTC region R1 in the ultrasound image U, as shown in Fig. 4 or Fig. 5. The association between the ultrasound image U and the category of the GTC region R1 in the training data can be performed by an expert, such as a skilled doctor.

The evaluation unit 29 can output, as the evaluation result, any one of a plurality of predetermined categories, for example, any one of two categories of Low and High as the category of the GTC region R1. Low indicates that the lobule atrophy has not progressed as much as in High. Further, the evaluation unit 29 can also output, for example, any one of four categories of Minimal, Mild, Moderate, and Marked as the category of the GTC region R1. Mild indicates that the atrophy of the lobule is not more advanced than that in Minimal, Moderate indicates that the atrophy of the lobule is not more advanced than that in Mild, and Marked indicates that the atrophy of the lobule is not more advanced than that in Moderate.

In a case of outputting the category of the GTC region R1 based on the ratio of the GTC region R1 in the mammary gland region M, the evaluation unit 29 can determine the category of the GTC region R1 based on any one of the average value, the median value, or the maximum value of the ratios of the predetermined number of GTC regions R1 obtained from the ultrasound images U of the predetermined number of frames, and use the category determined in this way as the final evaluation result. In addition, in a case in which a predetermined number of categories are output from the ultrasound images U of the predetermined number of frames using the trained model that has been trained through machine learning, the evaluation unit 29 can use a mode value of the predetermined number of categories as the final evaluation result.

It is generally known that both the GTC region R1 and the suspected lesion region A are depicted as the low-echo regions in the ultrasound image U. Therefore, for example, in a case in which the GTC evaluation is performed using the ultrasound image U of the frame in which the suspected lesion region A is detected, the GTC evaluation may be performed in a state in which the suspected lesion region A is regarded as the GTC region R1, and an accurate evaluation result may not be obtained. Since the evaluation unit 29 performs the GTC evaluation based on the evaluation target frame group G in which the suspected lesion region A is not detected, which is generated by the target frame generation unit 27, it is possible to improve the accuracy of the evaluation.

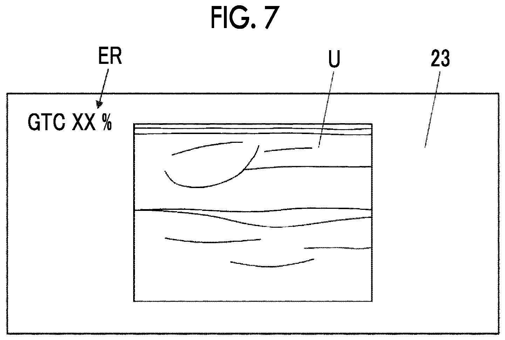

The display control unit 22 performs predetermined processing on the ultrasound image U transmitted from the image generation unit 21 under the control of the body control unit 31, and displays the ultrasound image U on the monitor 23. In addition, for example, as shown in Fig. 7, the display control unit 22 can display the evaluation result ER of the GTC evaluation output by the evaluation unit 29 and a representative ultrasound image U used for the GTC evaluation together on the monitor 23. In the example of Fig. 7, as the evaluation result ER of the GTC evaluation, the ratio of the GTC region R1 in the mammary gland region M is shown as a numerical value. In addition, as the representative ultrasound image U used for the GTC evaluation, for example, a latest ultrasound image U, an oldest ultrasound image U, or the ultrasound image U designated by the user via the input device 32 among the ultrasound images U of the predetermined number of frames used for the GTC evaluation can be displayed.

Even in a case in which there is the suspected lesion region A in the mammary gland region M of the subject, the user can accurately consider the risk of cancer in the mammary gland region M by confirming the evaluation result ER of the GTC evaluation displayed in this manner.

The monitor 23 displays the ultrasound image U and the like under the control of the display control unit 22, and includes, for example, a display device such as a liquid crystal display (LCD) or an organic electroluminescence display (organic EL display).

The evaluation result memory 30 stores the evaluation result ER of the GTC evaluation for each partial region performed by the evaluation unit 29. For example, the user can read out the evaluation result ER through the input device 32 after the examination of the subject and consider the risk of cancer in the breast of the subject based on the evaluation result ER. As the evaluation result memory 28, for example, recording media such as a flash memory, an HDD, an SSD, an FD, an MO disc, an MT, a RAM, a CD, a DVD, an SD card, and an USB memory can be used.

The body control unit 31 controls each unit of the apparatus body 2 and the transmission-and-reception circuit 12 of the ultrasound probe 1 based on a control program or the like, which is stored in advance.

The input device 32 is an input device used by the user to perform an input operation, and is configured by, for example, a device such as a keyboard, a mouse, a trackball, a touchpad, and a touch sensor disposed in a state of being superimposed on the monitor 23.

The processor 34 including the image generation unit 21, the display control unit 22, the mammary gland region extraction unit 25, the lesion detection unit 26, the target frame generation unit 27, the evaluation unit 29, and the body control unit 31 is configured by a central processing unit (CPU) and a control program for causing the CPU to execute various kinds of processing, but the processor 34 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the image generation unit 21, the display control unit 22, the mammary gland region extraction unit 25, the lesion detection unit 26, the target frame generation unit 27, the evaluation unit 29, and the body control unit 31 of the processor 34 can also be configured by being integrated partially or entirely into one CPU or the like.

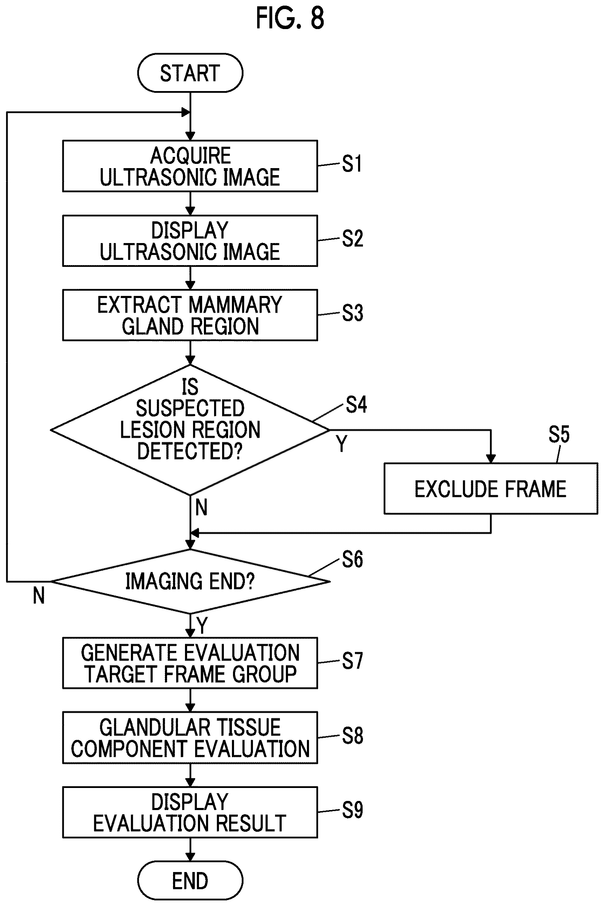

Hereinafter, an operation of the ultrasound diagnostic apparatus according to the embodiment will be described with reference to a flowchart shown in Fig. 8.

First, in step S1, the breast of the subject is imaged by using the ultrasound probe 1, and the ultrasound image U is acquired. In this case, under the control of the body control unit 31, the transmission and reception of the ultrasound waves from the plurality of transducers of the transducer array 11 are started in accordance with the drive signal from the pulser 13 of the transmission-and-reception circuit 12 of the ultrasound probe 1, the ultrasound echo from the inside of the breast of the subject is received by the plurality of transducers of the transducer array 11, the reception signal which is an analog signal is output to the amplifying unit 14 and is amplified by the amplifying unit 14, and the amplified reception signal is AD-converted by the AD conversion unit 15 to acquire the reception data.

The reception focus processing is performed on the reception data by the beam former 16, and the sound ray signal generated by the reception focus processing is transmitted to the image generation unit 21 of the apparatus body 2, and as a result, the ultrasound image U representing the tomographic image information of the breast of the subject is generated by the image generation unit 21. In this case, the signal processing unit 41 of the image generation unit 21 performs the correction of the attenuation in accordance with the depth of the reflection position of the ultrasound wave and the envelope detection processing on the sound ray signal, the DSC 42 performs the conversion into the image signal in accordance with the normal television signal scanning method, and the image processing unit 43 performs various types of necessary image processing such as gradation processing.

Then, in step S2, the ultrasound image U generated by the image generation unit 21 is displayed on the monitor 23 via the display control unit 22, and is stored in the image memory 24.

In a case of acquiring the ultrasound image U, the transmission intensity of the ultrasound wave and the depth range of the ultrasound image U displayed on the monitor 23 are adjusted under the control of the body control unit 31 such that the entire breast of the subject, that is, for example, the breast region BR of the subject shown in Fig. 4 or Fig. 5 is within the screen.

In subsequent step S3, the mammary gland region extraction unit 25 detects the breast region BR of the subject from the ultrasound image U acquired in step S1, and extracts the mammary gland region M from the detected breast region BR. The mammary gland region extraction unit 25 can perform the image recognition using at least one of template matching, an image analysis technique using a feature value, such as Adaboost, SVM, or SIFT, or a determination model that has been trained using a machine learning technique such as deep learning, in order to detect the breast region BR and to extract the mammary gland region M, for example.

In step S4, the lesion detection unit 26 performs processing of detecting the suspected lesion region A in the mammary gland region M extracted in step S3 based on the ultrasound image U acquired in step S1, and the target frame generation unit 27 determines whether or not the suspected lesion region A is detected by the lesion detection unit 26.

The lesion detection unit 26 performs processing of detecting the suspected lesion region A by using, for example, at least one of template matching, an image analysis technique using a feature value, such as Adaboost, SVM, or SIFT, or a determination model that has been trained by using a machine learning technique such as deep learning. In addition, the lesion detection unit 26 assigns the flag to the ultrasound image U of the frame in which the suspected lesion region A is detected. The target frame generation unit 27 can determine that the suspected lesion region A is detected in a case in which the flag is assigned to the ultrasound image U, and determine that the suspected lesion region A is not detected in a case in which the flag is not assigned to the ultrasound image U.

In a case in which the target frame generation unit 27 determines that the suspected lesion region A is detected, the processing proceeds to step S5.

In step S5, the target frame generation unit 27 excludes the ultrasound image U in which the suspected lesion region A is detected in step S4, from the candidates of the evaluation target frame group G.

In addition, in a case in which it is determined in step S4 that the suspected lesion region A is not detected, the target frame generation unit 27 leaves the ultrasound image U acquired in step S1 as the candidate of the evaluation target frame group G.

In a case in which the processing of step S5 is completed or the target frame generation unit 27 determines that the suspected lesion region A is not detected, the processing proceeds to step S6. In step S6, the body control unit 31 determines whether or not to end the capture of the ultrasound image U. The body control unit 31 can determine to end the capture of the ultrasound image U, for example, in a case in which an instruction to end the imaging is input by the user via the input device 32, and determine to continue the capture of the ultrasound image U in a case in which the instruction to end the imaging is not particularly input by the user via the input device 32.

In a case in which it is determined in step S6 that the capture of the ultrasound image U is continued, the processing returns to step S1, the ultrasound image U is newly acquired, and then the processing of step S2 to the processing of step S6 are sequentially performed. In this way, as long as it is determined in step S6 to continue the capture of the ultrasound image U, the processing of step S1 to the processing of step S6 are repeated, and the ultrasound images U of the plurality of frames are left as the candidates of the evaluation target frame group G.

In step S7 following step S6, the target frame generation unit 27 generates the evaluation target frame group G with the ultrasound images U of the plurality of frames for which it is determined that the suspected lesion region A is not detected in the repetition of step S1 to step S6.

In step S8, the evaluation unit 29 extracts the GTC region R1 from the mammary gland region M extracted in step S3, calculates the ratio of the GTC region R1 in the mammary gland region M, and performs the GTC evaluation, based on the calculated ratio of the GTC region R1, on the ultrasound image U of each frame in the evaluation target frame group G generated in step S7.

The evaluation unit 29 can distinguish between the GTC region R1 and the edematous stroma R2 in the mammary gland region M by, for example, binarizing the mammary gland region M of the ultrasound image U using the brightness threshold value Thb, and can extract the GTC region R1. In addition, the evaluation unit 29 can extract the GTC region R1 using the trained model that has learned the GTC region R1 (segmentation) in the mammary gland region M in the training ultrasound image in which the breast is imaged, in machine learning such as deep learning.

The evaluation unit 29 can calculate the ratio of the GTC region R1, for example, by the ratio of the sum of the number of pixels occupied by all the low-echo regions in the mammary gland region M to the number of pixels occupied by the high-echo region in the mammary gland region M in the ultrasound image U. The evaluation unit 29 can output, for example, the average value, the median value, or the maximum value of the ratios of the GTC region R1 in the mammary gland region M calculated for the ultrasound images U of the plurality of frames in this way as the final evaluation result ER.

In addition, the evaluation unit 29 can also determine the category of the GTC region R1 based on, for example, the average value, the median value, or the maximum value of the ratios of the GTC region R1 in the mammary gland region M, which is calculated for the ultrasound images U of the plurality of frames, and output the category as the evaluation result ER. In this case, the evaluation unit 29 can output any one of a plurality of predetermined categories, for example, the two categories of Low and High, or the four categories of Minimal, Mild, Moderate, and Marked, as the category of the GTC region R1.

In addition, the evaluation unit 29 can output the category of the GTC region R1 for each of the ultrasound images U of the plurality of frames by inputting the ultrasound images U of the plurality of frames to the trained model that has been trained through machine learning, and output the mode value as the final evaluation result ER of the GTC evaluation.

In general, it is known that both the GTC region R1 and the suspected lesion region A are depicted as low-echo regions in the ultrasound image U, and in a case in which the GTC evaluation is performed in a state in which the suspected lesion region A is regarded as the GTC region R1, an accurate evaluation result ER may not be obtained, but since the evaluation unit 29 performs the GTC evaluation based on the evaluation target frame group G in which the suspected lesion region A is not detected, a highly accurate evaluation result ER can be output.

At last, in step S9, the display control unit 22 displays the evaluation result ER of the GTC evaluation output in step S7 on the monitor 23, for example, as shown in Fig. 7.

Since the user can ascertain an accurate evaluation result ER by confirming this display, the user can accurately consider the risk of cancer in the breast of the subject even in a case in which the suspected lesion region A is present in the mammary gland region M.

In a case in which the processing of step S9 is completed in this manner, the operation of the ultrasound diagnostic apparatus according to the flowchart of Fig. 8 is completed.

As described above, with the ultrasound diagnostic apparatus according to Embodiment 1, the lesion detection unit 26 detects the suspected lesion region A in the mammary gland region M for each of the ultrasound images U of the plurality of frames, the target frame generation unit 27 generates the evaluation target frame group G with the ultrasound images U of the frames other than the frame in which the suspected lesion region A is detected by the lesion detection unit 26 among the ultrasound images U of the plurality of frames, and the evaluation unit 29 performs the GTC evaluation on the ultrasound images U of each frame in the evaluation target frame group G, so that the user can accurately consider the risk of cancer in the mammary gland region M of the subject even in a case in which the suspected lesion region A is present.

In addition, a case has been described in which the transmission-and-reception circuit 12 is provided in the ultrasound probe 1, but the transmission-and-reception circuit 12 may be provided in the apparatus body 2.

A case has been described in which the image generation unit 21 is provided in the apparatus body 2, but the image generation unit 21 may be provided in the ultrasound probe 1.

The apparatus body 2 may be a so-called stationary type, a portable type that is easy to carry, or a so-called handheld type that is configured by, for example, a smartphone or a tablet type computer. As described above, the type of the device constituting the apparatus body 2 is not particularly limited.

A case has been described in which the ultrasound probe 1 and the apparatus body 2 are connected to each other in a wired manner, but the ultrasound probe 1 and the apparatus body 2 may be connected to each other in a wireless manner.

In addition, the target frame generation unit 27 can generate the evaluation target frame group G with the ultrasound images U of the frames on and before a frame that is a predetermined number of frames before the frame in which the suspected lesion region A is detected by the lesion detection unit 26 and the ultrasound images U of the frames on and after a frame that is a predetermined number of frames after the frame in which the suspected lesion region A is detected by the lesion detection unit 26, among the ultrasound images U of the plurality of frames.

For example, in a case in which the suspected lesion region A is detected in the ultrasound image U of the N-th frame among the ultrasound images U of the plurality of frames, the target frame generation unit 27 can set a predetermined number of frames to X, generate the evaluation target frame group G with the ultrasound images U of the frames on and before an (N-X)-th frame and the ultrasound images U of the frames on and after an (N+X)-th frame, and exclude the ultrasound images U of the frames from the (N-X+1)-th frame to the (N+X-1)-th frame from the evaluation target frame group G. Here, X is an integer of 0 or more, N is a natural number of 2 or more, and X < N is satisfied.

In the ultrasound images U of several frames before and after the frame in which the suspected lesion region A is detected, even in a case in which the suspected lesion region A is not detected by the lesion detection unit 26, it is considered that the ultrasound images U actually include the suspected lesion region A due to some reason such as the ultrasound images U being unclear. Therefore, by generating the evaluation target frame group G with the ultrasound images U of the frames on and before the frame that is a predetermined number of frames before the frame in which the suspected lesion region A is detected and the ultrasound images U of the frames on and after the frame that is a predetermined number of frames after the frame in which the suspected lesion region A is detected by the lesion detection unit 26, the evaluation unit 29 can more reliably exclude the ultrasound image U including the suspected lesion region A from the evaluation target frame group G and accurately perform the GTC evaluation.

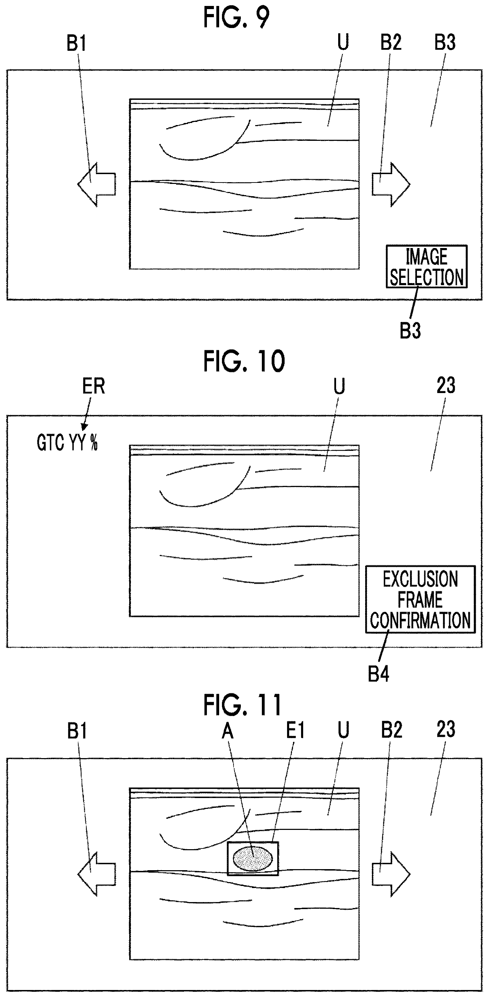

In addition, for example, the display control unit 22 can display the ultrasound image U of each frame in the evaluation target frame group G generated by the target frame generation unit 27 on the monitor 23, such as a display screen of the monitor 23 as shown in Fig. 9. In the example of Fig. 9, a first arrow button B1 facing a left direction, a second arrow button B2 facing a right direction, the ultrasound image U, and an image selection button B3 are displayed on the display screen of the monitor 23. For example, the user selects the first arrow button B1 and the second arrow button B2 via the input device 32, and the ultrasound image U of each frame IN the evaluation target frame group G is switched one frame at a time and displayed on the monitor 23.

In this case, the user can designate the ultrasound image U currently displayed on the monitor 23 by selecting the image selection button B3 via, for example, the input device 32. The evaluation unit 29 can perform the GTC evaluation on the ultrasound image U of the frame designated by the user in this manner among the evaluation target frame group G. As a result, the user can confirm not only the evaluation result ER of the GTC evaluation based on the ultrasound images U of the plurality of frames but also the evaluation result ER of the GTC evaluation for the ultrasound image U of the specific frame desired by the user, and can more specifically consider the risk of cancer in the mammary gland region M.

In addition, the display control unit 22 can also display the ultrasound image U of the frame in which the suspected lesion region A is detected by the lesion detection unit 26 among the ultrasound images U of the plurality of frames, on the monitor 23. In this case, first, the display control unit 22 can perform display as shown in Fig. 10 on the monitor 23. In the example of Fig. 10, the evaluation result ER of the GTC evaluation, the representative ultrasound image U in the evaluation target frame group G, and an exclusion frame confirmation button B4 are displayed on the monitor 23.

The display control unit 22 can perform display as shown in Fig. 11 on the monitor 23, for example, in response to the user selecting the exclusion frame confirmation button B4 via the input device 32. In the example of Fig. 11, the first arrow button B1, the second arrow button B2, and the ultrasound image U in which the suspected lesion region A is shown are displayed on the display screen of the monitor 23. For example, the user selects the first arrow button B1 and the second arrow button B2 via the input device 32, and the ultrasound image U of each frame IN the evaluation target frame group G is switched one frame at a time and displayed on the monitor 23.

In this case, the display control unit 22 can highlight the suspected lesion region A, for example, by superimposing a frame line E1 surrounding the suspected lesion region A on the ultrasound image U so that the user can easily confirm the suspected lesion region A. The method of highlighting the suspected lesion region A is not particularly limited, and for example, any other method of displaying the frame line E1, such as giving a color different from the surroundings to the suspected lesion region A, blinking the suspected lesion region A, or highlighting and displaying a contour line of the suspected lesion region A, can also be used.

In some cases, the lesion detection unit 26 may not be able to normally detect the suspected lesion region A due to some reason, such as the ultrasound image U being unclear. Therefore, the display control unit 22 can display, on the monitor 23, a dialog for confirming with the user whether or not to correct the detection result of the suspected lesion region A obtained by the lesion detection unit 26 for the ultrasound image U of each frame for which the processing of detecting the suspected lesion region A is performed by the lesion detection unit 26. The user can confirm the dialog and then correct the detection result of the suspected lesion region A by the input operation via the input device 32.

Although it has been described that the evaluation result ER of the GTC evaluation output by the evaluation unit 29 is stored in the evaluation result memory 30, the evaluation result ER can also be stored in association with the ultrasound image U used for the GTC evaluation.

The apparatus body 2 can also comprise a transmission circuit (not shown) that transmits the evaluation result ER of the GTC evaluation output by the evaluation unit 29 to an external server device (not shown) such as an examination information management system such as a so-called electronic medical record, a report system that creates a report using a medical image, and a picture archiving and communication system (PACS). In a case of transmitting the evaluation result ER to the external server device or the like, for example, a protocol, such as hypertext transfer protocol (HTTP), hypertext transfer protocol secure (HTTPS), file transfer protocol (FTP), health level seven (HL7), or digital imaging and communications in medicine (DICOM), can be used.

### [Embodiment 2]

The target frame generation unit 27 can also determine the ultrasound image U to be included in the evaluation target frame group G in consideration of a size of the suspected lesion region A detected by the lesion detection unit 26.

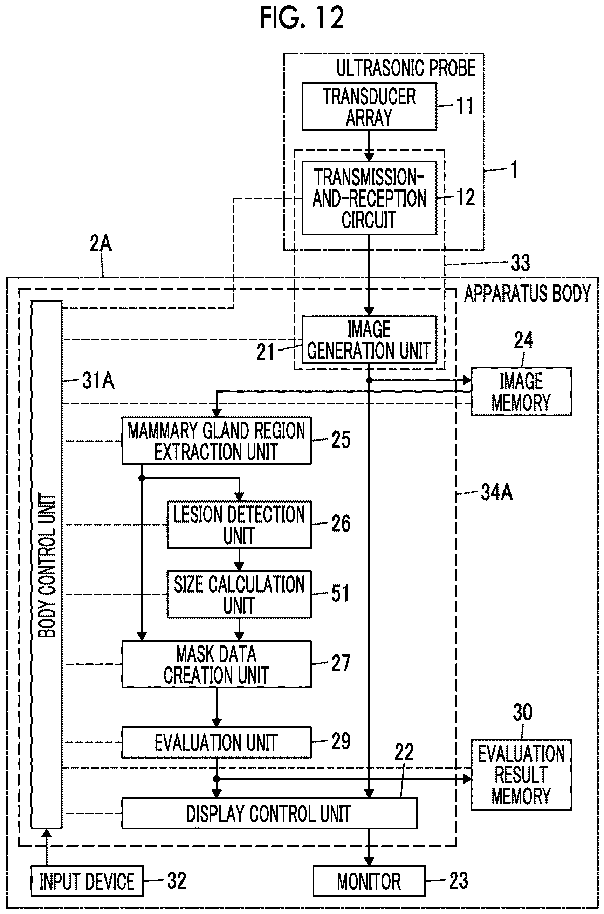

Fig. 12 shows a configuration of an ultrasound diagnostic apparatus according to Embodiment 2. The ultrasound diagnostic apparatus according to Embodiment 2 comprises an apparatus body 2A instead of the apparatus body 2 in the ultrasound diagnostic apparatus according to Embodiment 1 shown in Fig. 1. The apparatus body 2A newly comprises a size calculation unit 51 and comprises a body control unit 31A instead of the body control unit 31, as compared to the apparatus body 2 according to Embodiment 1.

In the apparatus body 2A, the size calculation unit 51 is connected to the lesion detection unit 26. The size calculation unit 51 is connected to the target frame generation unit 27 and the body control unit 31A. In addition, the image generation unit 21, the display control unit 22, the mammary gland region extraction unit 25, the lesion detection unit 26, the target frame generation unit 27, the evaluation unit 29, the body control unit 31A, and the size calculation unit 51 constitute a processor 34A for the apparatus body 2A.

The size calculation unit 51 calculates the size of the suspected lesion region A detected by the lesion detection unit 26. The size calculation unit 51 can calculate, for example, a maximum dimension of the suspected lesion region A or the number of pixels occupied by the suspected lesion region A in the mammary gland region M as the size of the suspected lesion region A.

Here, in a case in which the size of the detected suspected lesion region A is extremely small, even in a case in which the ultrasound image U including the suspected lesion region A is included in the evaluation target frame group G, the evaluation result ER equivalent to the evaluation result ER of the GTC evaluation in a case in which the ultrasound image U is excluded from the evaluation target frame group G is obtained, so that the target frame generation unit 27 can include, in the evaluation target frame group G, the ultrasound image U of the frame in which the size of the suspected lesion region A calculated by the size calculation unit 51 is less than the predetermined size threshold value.

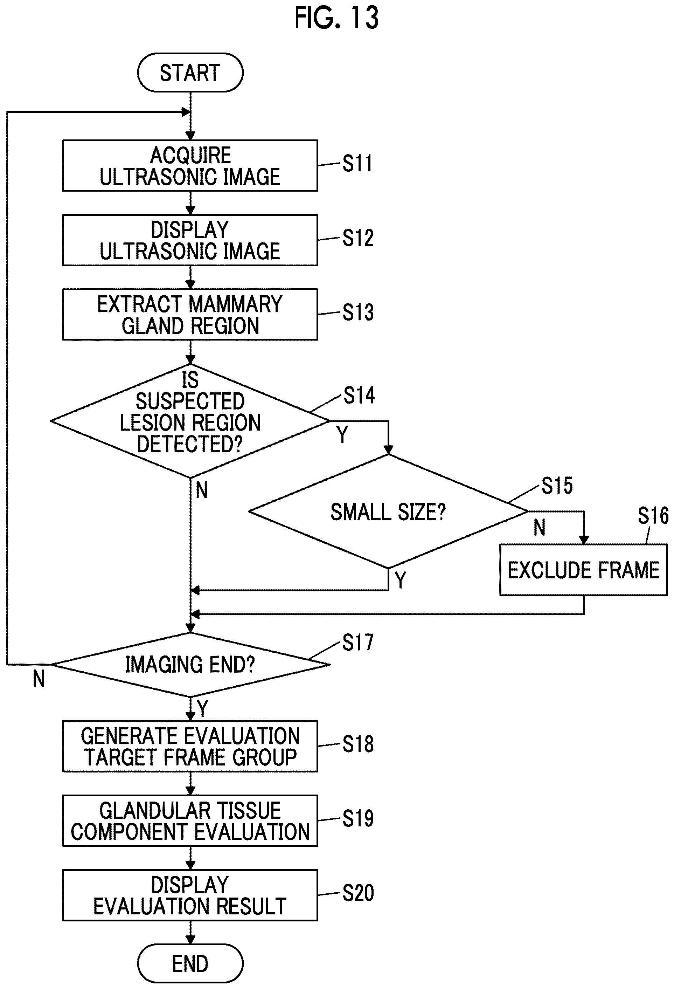

Next, an operation of the ultrasound diagnostic apparatus according to Embodiment 2 will be described with reference to the flowchart of Fig. 13. The flowchart of Fig. 13 is obtained by adding step S15 to the flowchart of Embodiment 1 shown in Fig. 8, step S11 to step S14 correspond to step S1 to step S4 shown in Fig. 8, and step S16 to step S20 correspond to step S5 to step S9 shown in Fig. 8. Therefore, steps S11 to S14 and steps S16 to S20 will not be described in detail.

In a case in which the ultrasound image U is acquired in step S11, the ultrasound image U is displayed on the monitor 23 in step S12, and the mammary gland region M is extracted from the ultrasound image U in step S13, the processing proceeds to step S14.

In step S14, the lesion detection unit 26 performs processing of detecting the suspected lesion region A from the ultrasound image U, and the target frame generation unit 27 determines whether or not the suspected lesion region A is detected by this processing. Here, in a case in which it is determined that the suspected lesion region A is detected, the processing proceeds to step S15.

In step S15, the size calculation unit 51 calculates the size of the suspected lesion region A detected in step S14, and the target frame generation unit 27 determines whether or not the calculated size of the suspected lesion region A is less than a predetermined size threshold value. In this case, the size calculation unit 51 can calculate, for example, the maximum dimension of the suspected lesion region A or the number of pixels occupied by the suspected lesion region A in the mammary gland region M as the size of the suspected lesion region A.

In a case in which it is determined in step S15 that the size of the suspected lesion region A is equal to or greater than the predetermined size threshold value, the processing proceeds to step S16. In step S16, the target frame generation unit 27 excludes the ultrasound image U acquired in step S11 from the candidates of the evaluation target frame group G.

In a case in which it is determined in step S15 that the size of the suspected lesion region A is less than the predetermined size threshold value, the target frame generation unit 27 leaves the ultrasound image U acquired in step S11 as the candidate of the evaluation target frame group G.

In a case in which it is determined in step S14 that the suspected lesion region A is not detected, in a case in which it is determined in step S15 that the size of the suspected lesion region A is less than the predetermined size threshold value, or in a case in which the processing of step S16 is completed, the processing proceeds to step S17. In step S17, the body control unit 31A determines whether or not to end the capture of the ultrasound image U.

As long as it is determined to continue the capture of the ultrasound image U in step S17, the processing of step S11 to the processing of step S17 is repeated. In a case in which it is determined in step S17 to end the capture of the ultrasound image U, the processing proceeds to step S18, and the evaluation target frame group G is generated by the target frame generation unit 27. The evaluation target frame group G generated here includes the ultrasound image U in which the suspected lesion region A having a size less than a predetermined size threshold value is shown, but the suspected lesion region A is very small, and thus there is almost no adverse effect on the GTC evaluation.

In step S19, the evaluation unit 29 performs the GTC evaluation by using the evaluation target frame group G generated in step S18. In subsequent step S20, the display control unit 22 displays the evaluation result ER of the GTC evaluation output in step S19 on the monitor 23, for example, as shown in Fig. 7.

In a case in which the processing of step S20 is completed in this manner, the operation of the ultrasound diagnostic apparatus according to the flowchart of Fig. 13 is completed.

As described above, in the ultrasound diagnostic apparatus according to Embodiment 2, the size calculation unit 51 calculates the size of the suspected lesion region A detected by the lesion detection unit 26, and the target frame generation unit 27 includes, in the evaluation target frame group G, the ultrasound image U of the frame in which the size of the suspected lesion region A calculated by the size calculation unit 51 is less than the predetermined size threshold value, but an accurate evaluation result ER of the GTC evaluation can be obtained as in the ultrasound diagnostic apparatus according to Embodiment 1, so that the user can accurately consider the risk of cancer in the mammary gland region M of the subject even in a case in which the suspected lesion region A is present.

### [Embodiment 3]

The target frame generation unit 27 can also determine the ultrasound image U to be included in the evaluation target frame group G in consideration of a malignancy degree of the suspected lesion region A detected by the lesion detection unit 26.

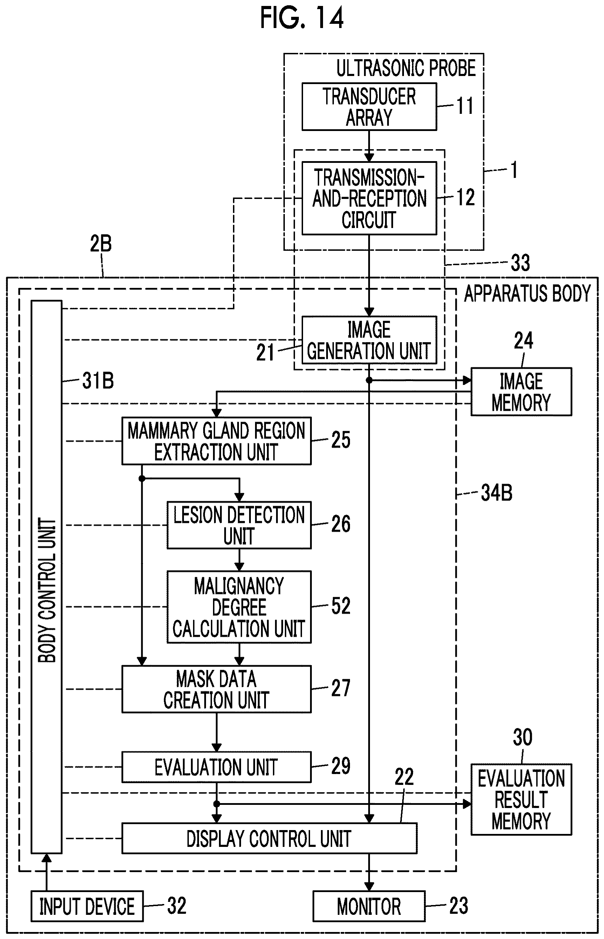

Fig. 14 shows a configuration of an ultrasound diagnostic apparatus according to Embodiment 3. The ultrasound diagnostic apparatus according to Embodiment 3 comprises an apparatus body 2B instead of the apparatus body 2 with respect to the ultrasound diagnostic apparatus according to Embodiment 1 shown in Fig. 1. The apparatus body 2B newly comprises a malignancy degree calculation unit 52 and comprises a body control unit 31B instead of the body control unit 31, as compared to the apparatus body 2 according to Embodiment 1.

In the apparatus body 2B, the malignancy degree calculation unit 52 is connected to the lesion detection unit 26. The malignancy degree calculation unit 52 is connected to the target frame generation unit 27 and the body control unit 31B. In addition, the image generation unit 21, the display control unit 22, the mammary gland region extraction unit 25, the lesion detection unit 26, the target frame generation unit 27, the evaluation unit 29, the body control unit 31B, and the malignancy degree calculation unit 52 constitute a processor 34B for the apparatus body 2B.

The malignancy degree calculation unit 52 calculates the malignancy degree of the suspected lesion region A detected by the lesion detection unit 26, for example, by performing image analysis.

Here, for example, in general, it is known that benign tumors often have a circular or elliptical shape, and malignant tumors such as breast cancer often have a so-called lobed or polygonal shape. The malignancy degree calculated by the malignancy degree calculation unit 52 refers to a probability that the tissue in the set suspected lesion region A is malignant. For example, the higher the malignancy degree of the pixel, the higher the probability that the pixel represents a tissue in a malignant lesion part, and the lower the malignancy degree of the pixel, the higher the probability that the pixel represents a tissue in a benign lesion part.

The malignancy degree calculation unit 52 can calculate the malignancy degree of the suspected lesion region A by recognizing the shape of the tissue using, for example, a method of image recognition including pattern matching and extraction of a so-called feature value, a deep learning method, or the like. In a case in which the deep learning method is used, the malignancy degree calculation unit 52 can calculate the malignancy degree by learning a plurality of ultrasound images including malignant tumors and a plurality of ultrasound images including benign tumors as so-called training data in advance and comparing a relationship between the brightness of a specific pixel in the suspected lesion region A and the brightness of the surrounding pixels with the learned data.

The target frame generation unit 27 can include, in the evaluation target frame group G, the ultrasound image U of the frame in which the malignancy degree of the suspected lesion region A calculated by the malignancy degree calculation unit 52 is less than a predetermined malignancy degree threshold value.

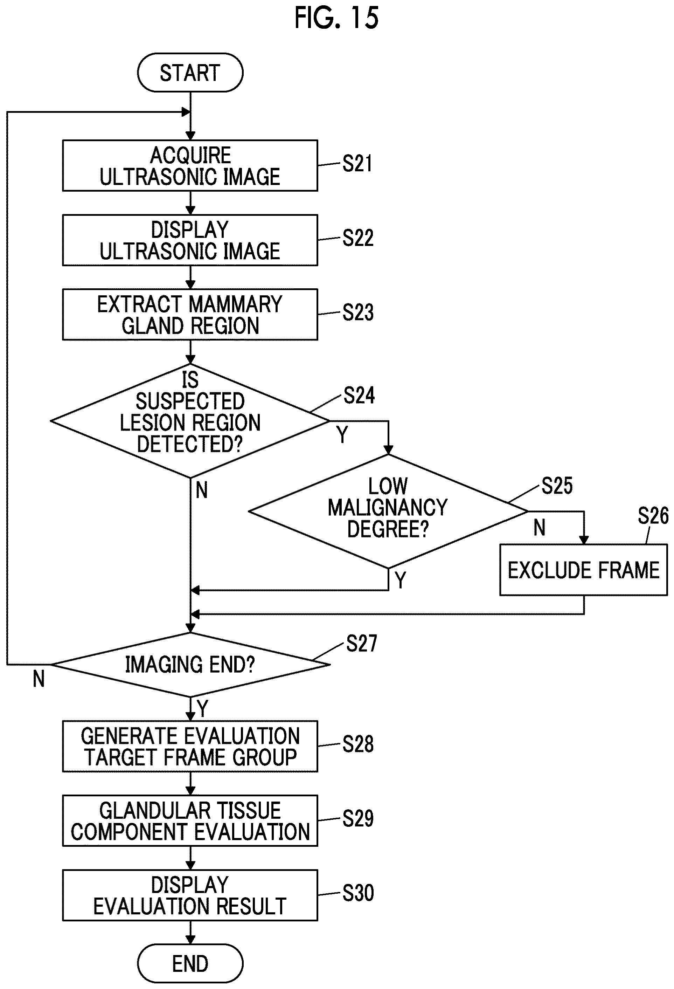

Next, an operation of the ultrasound diagnostic apparatus according to Embodiment 3 will be described with reference to the flowchart of Fig. 15. The flowchart of Fig. 15 is obtained by adding step S25 to the flowchart of Embodiment 1 shown in Fig. 8, step S21 to step S24 correspond to step S1 to step S4 shown in Fig. 8, and step S26 to step S30 correspond to step S5 to step S9 shown in Fig. 8. Therefore, step S21 to step S24 and step S26 to step S30 will not be described in detail.

In a case in which the ultrasound image U is acquired in step S21, the ultrasound image U is displayed on the monitor 23 in step S22, and the mammary gland region M is extracted from the ultrasound image U in step S23, the processing proceeds to step S24.

In step S24, the lesion detection unit 26 performs processing of detecting the suspected lesion region A from the ultrasound image U, and the target frame generation unit 27 determines whether or not the suspected lesion region A is detected by this processing. Here, in a case in which it is determined that the suspected lesion region A is detected, the processing proceeds to step S25.

In step S25, the malignancy degree calculation unit 52 calculates the malignancy degree of the suspected lesion region A detected in step S24, and the target frame generation unit 27 determines whether or not the calculated malignancy degree of the suspected lesion region A is less than a predetermined malignancy degree threshold value.

The malignancy degree calculation unit 52 can calculate the malignancy degree of the suspected lesion region A by recognizing the shape of the tissue using, for example, a method of image recognition including pattern matching and extraction of a so-called feature value, a deep learning method, or the like.

In a case in which it is determined in step S25 that the malignancy degree of the suspected lesion region A is equal to or greater than the predetermined malignancy degree threshold value, the processing proceeds to step S26. In step S26, the target frame generation unit 27 excludes the ultrasound image U acquired in step S21 from the candidates of the evaluation target frame group G.

In a case in which it is determined in step S25 that the malignancy degree of the suspected lesion region A is less than the predetermined malignancy degree threshold value, the target frame generation unit 27 leaves the ultrasound image U acquired in step S21 as the candidate of the evaluation target frame group G.

In a case in which it is determined in step S24 that the suspected lesion region A is not detected, in a case in which it is determined in step S25 that the malignancy degree of the suspected lesion region A is less than the predetermined malignancy degree threshold value, or in a case in which the processing of step S26 is completed, the processing proceeds to step S27. In step S27, the body control unit 31B determines whether or not to end the capture of the ultrasound image U.

The processing of step S21 to the processing of step S27 are repeated as long as it is determined to continue the capture of the ultrasound image U in step S27. In a case in which it is determined in step S27 to end the capture of the ultrasound image U, the processing proceeds to step S28, and the evaluation target frame group G is generated by the target frame generation unit 27. The evaluation target frame group G generated here does not include the ultrasound image U in which the suspected lesion region A with the malignancy degree greater than the predetermined malignancy degree threshold value, that is, the suspected lesion region A that can be sufficiently determined to be malignant is shown.

In step S29, the evaluation unit 29 performs the GTC evaluation by using the evaluation target frame group G generated in step S28. Since the evaluation target frame group G does not include the suspected lesion region A that can be sufficiently determined to be malignant, the evaluation unit 29 can accurately perform the GTC evaluation. In subsequent step S30, the display control unit 22 displays the evaluation result ER of the GTC evaluation output in step S29 on the monitor 23, for example, as shown in Fig. 7.

In a case in which the processing of step S30 is completed in this manner, the operation of the ultrasound diagnostic apparatus according to the flowchart of Fig. 15 is completed.

As described above, in the ultrasound diagnostic apparatus according to Embodiment 3, the malignancy degree calculation unit 52 calculates the malignancy degree of the suspected lesion region A detected by the lesion detection unit 26, and the target frame generation unit 27 includes, in the evaluation target frame group G, the ultrasound image U of the frame in which the size of the suspected lesion region A calculated by the malignancy degree calculation unit 52 is less than the predetermined malignancy degree threshold value, so that the evaluation unit 29 can accurately perform the GTC evaluation, and the user can accurately consider the risk of cancer in the mammary gland region M of the subject even in a case in which the suspected lesion region A is present.

The ultrasound diagnostic apparatus according to Embodiment 3 has a configuration in which the malignancy degree calculation unit 52 is added to the ultrasound diagnostic apparatus according to Embodiment 1, but the ultrasound diagnostic apparatus according to Embodiment 3 can also have a configuration in which the malignancy degree calculation unit 52 is added to the ultrasound diagnostic apparatus according to Embodiment 2.

### Explanation of References

1: ultrasound probe
2, 2A, 2B: apparatus body
11: transducer array
12: transmission-and-reception circuit
13: pulser
14: amplifying unit
15: AD conversion unit
16: beam former
21: image generation unit
22: display control unit
23: monitor
24: image memory
25: mammary gland region extraction unit
26: lesion detection unit
27: mask data creation unit
28: exclusion region setting unit
29: evaluation unit
30: evaluation result memory
31, 31A, 31B: body control unit
32: input device
33: image acquisition unit
34, 34A, 34B: processor
41: signal processing unit
42: DSC
43: image processing unit
51: size calculation unit
52: malignancy degree calculation unit
A: suspected lesion region
B1: first arrow button
B2: second arrow button
B3: image selection button
B4: exclusion frame confirmation button
BR: breast region
E1: frame line
ER: evaluation result
G: evaluation target frame group
L1: front boundary line
L2: rear boundary line
M: mammary gland region
R1: GTC region
S: skin
T: pectoralis major
U: ultrasound image

## Claims

1. An ultrasound diagnostic apparatus comprising:
an image acquisition unit that continuously acquires ultrasound images of a plurality of frames in which a mammary gland region of a subject is imaged;
a lesion detection unit that detects a suspected lesion region in the mammary gland region for each of the ultrasound images of the plurality of frames;
a target frame generation unit that generates an evaluation target frame group with ultrasound images of frames other than a frame in which the suspected lesion region is detected by the lesion detection unit among the ultrasound images of the plurality of frames; and
an evaluation unit that performs a glandular tissue component evaluation on the ultrasound image of each frame in the evaluation target frame group.

2. The ultrasound diagnostic apparatus according to claim 1,
wherein the target frame generation unit generates the evaluation target frame group with ultrasound images of frames on and before a frame that is a predetermined number of frames before the frame in which the suspected lesion region is detected by the lesion detection unit and ultrasound images of frames on and after a frame that is a predetermined number of frames after the frame in which the suspected lesion region is detected by the lesion detection unit, among the ultrasound images of the plurality of frames.

3. The ultrasound diagnostic apparatus according to claim 1, further comprising:
a size calculation unit that calculates a size of the suspected lesion region detected by the lesion detection unit,
wherein the target frame generation unit includes, in the evaluation target frame group, an ultrasound image of a frame in which the size of the suspected lesion region calculated by the size calculation unit is less than a predetermined size threshold value.

4. The ultrasound diagnostic apparatus according to claim 1, further comprising:
a malignancy degree calculation unit that calculates a malignancy degree of the suspected lesion region detected by the lesion detection unit,
wherein the target frame generation unit includes, in the evaluation target frame group, an ultrasound image of a frame in which the malignancy degree of the suspected lesion region calculated by the malignancy degree calculation unit is less than a predetermined malignancy threshold value.

5. The ultrasound diagnostic apparatus according to claim 1, further comprising:
a monitor; and
a display control unit that displays the plurality of ultrasound images on the monitor.

6. The ultrasound diagnostic apparatus according to claim 5,
wherein the display control unit displays the ultrasound image of each frame in the evaluation target frame group generated by the target frame generation unit, on the monitor.

7. The ultrasound diagnostic apparatus according to claim 6,
wherein the evaluation unit performs the glandular tissue component evaluation on an ultrasound image of a frame designated by a user in the evaluation target frame group.

8. The ultrasound diagnostic apparatus according to claim 5,
wherein the display control unit displays an ultrasound image of the frame in which the suspected lesion region is detected by the lesion detection unit among the ultrasound images of the plurality of frames, on the monitor.

9. The ultrasound diagnostic apparatus according to claim 8,
wherein the display control unit highlights the suspected lesion region on the monitor.

10. The ultrasound diagnostic apparatus according to claim 5,
wherein the display control unit displays an ultrasound image of each frame in which processing of detecting the suspected lesion region is performed by the lesion detection unit, on the monitor.

11. The ultrasound diagnostic apparatus according to claim 10,
wherein the display control unit displays a dialog for confirming with a user whether or not to correct a detection result of the suspected lesion region obtained by the lesion detection unit, on the monitor.

12. The ultrasound diagnostic apparatus according to claim 5,
wherein the evaluation unit classifies the mammary gland region of the ultrasound image of each frame in the evaluation target frame group into a low-echo region and a high-echo region based on a predetermined brightness threshold value, and displays a glandular tissue component ratio represented by a ratio between the number of pixels occupied by the low-echo region and the number of pixels occupied by the high-echo region, on the monitor as a result of the glandular tissue component evaluation.

13. The ultrasound diagnostic apparatus according to claim 12,
wherein the evaluation unit displays any one of an average value, a median value, or a maximum value of the glandular tissue component ratios calculated in each frame in the evaluation target frame group, or any one of an average value, a median value, or a maximum value of the glandular tissue component ratios obtained by excluding outliers from the glandular tissue component ratios calculated in each frame in the evaluation target frame group, on the monitor as the result of the glandular tissue component evaluation.

14. The ultrasound diagnostic apparatus according to claim 5,
wherein the evaluation unit determines a category of a glandular tissue component in the mammary gland region based on the ultrasound images in the evaluation target frame group, and displays the category on the monitor as a result of the glandular tissue component evaluation.

15. The ultrasound diagnostic apparatus according to claim 1,
wherein the lesion detection unit detects the suspected lesion region using a trained model that has been trained through machine learning based on a plurality of training data each of which includes the ultrasound image in which the mammary gland region including the suspected lesion region is imaged.

16. The ultrasound diagnostic apparatus according to claim 1,
wherein the lesion detection unit detects the suspected lesion region by image-analyzing the ultrasound image.

17. A method of controlling an ultrasound diagnostic apparatus, the method comprising:
continuously acquiring ultrasound images of a plurality of frames in which a mammary gland region of a subject is imaged;
detecting a suspected lesion region in the mammary gland region for each of the ultrasound images of the plurality of frames;
generating an evaluation target frame group with ultrasound images of frames other than a frame in which the suspected lesion region is detected among the ultrasound images of the plurality of frames; and
performing a glandular tissue component evaluation on the ultrasound image of each frame in the evaluation target frame group.
